# EUROPEAN PATENT SPECIFICATION

(11) **EP 2 970 958 B1**
(45) Date of publication and mention of the grant of the patent: **06.12.2017**
(21) Application number: 14722886.0
(22) Date of filing: 14.03.2014
(51) Int. Cl.: C12N 15/10, C12Q 1/68

(54) **METHODS OF SEQUENCING THE IMMUNE REPERTOIRE**
VERFAHREN ZUR SEQUENZIERUNG DES IMMUNREPERTOIRS
MÉTHODE DE SÉQUENÇAGE DU RÉPERTOIRE IMMUNITAIRE

(30) Priority: 15.03.2013 US 201361801785 P; 28.03.2013 US 201361806143 P
(43) Date of publication of application: 20.01.2016
(73) Proprietor: LINEAGE BIOSCIENCES, INC., Vancouver, BC V6T 1Z4 (CA)
(72) Inventor: HUTCHINS, Edward, A., Mountain View, CA 94040 (US); FAN, Hei-Mun, Christina, Fremont, CA 94538 (US)
(74) Representative: Patent Boutique LLP
(86) International application number: PCT/US2014/029241
(87) International publication number: WO 2014/144713

(56) References cited:
- WO-A1-2004/024953
- WO-A1-2005/019452
- WO-A1-2005/059176
- WO-A2-97/24455
- WO-A2-2009/152928
- WO-A2-2011/140433
- WO-A2-2012/083069
- JIANG NING ET AL: "Lineage structure of the human antibody repertoire in response to influenza vaccination.", SCIENCE TRANSLATIONAL MEDICINE 6 FEB 2013, vol. 5, no. 171, 6 February 2013 (2013-02-06), page 171ra19, XP009180301, ISSN: 1946-6242
- GUIDO SCHRAMM ET AL: "A simple and reliable 5'-RACE approach", NUCLEIC ACIDS RESEARCH, OXFORD UNIVERSITY PRESS, GB, vol. 28, no. 22, 15 November 2000 (2000-11-15), pages 1-4, XP002661255, ISSN: 1362-4962
- WANG ENA ET AL: "high-fidelity mRNA amplification for gene profiling", NATURE BIOTECHNOLOGY, NATURE PUBLISHING GROUP, NEW YORK, NY, US, vol. 18, 1 April 2000 (2000-04-01), pages 457-459, XP002211679, ISSN: 1087-0156, DOI: 10.1038/74546
- GEORGIOU GEORGE ET AL: "The promise and challenge of high-throughput sequencing of the antibody repertoire.", NATURE BIOTECHNOLOGY FEB 2014, vol. 32, no. 2, February 2014 (2014-02), pages 158-168, XP002730089, ISSN: 1546-1696
- HUBERT HUG ET AL: "Measurement of the number of molecules of a single mRNA species in a complex mRNA preparation.", JOURNAL OF THEORETICAL BIOLOGY, vol. 221, no. 4, 1 April 2003 (2003-04-01) , pages 615-624, XP55017448, ISSN: 0022-5193

## Description

### FIELD OF THE INVENTION

The present invention relates to the field of quantitative nucleic acid analysis. More specifically, the present invention provides methods of determining the immune repertoire using high throughput sequencing.

### BACKGROUND

A feature of the adaptive immune response is the ability to generate a wide diversity of binding molecules, e.g. T-cell antigen receptors and antibodies. A variety of molecular mechanisms exist to generate initial diversity, including genetic recombination at multiple sites. Armed with this initial repertoire of binding moieties, naive B and T-cells circulate where they can come in contact with antigen. Upon exposure to antigen there can be a positive selection process, where cells expressing immunological receptors having desired binding properties are expanded, and may undergo further sequence modification, for example somatic hypermutation, and additional recombination. There can also be a negative selection process, where cells expressing immunological receptors having undesirable binding properties, such as self-reactivity, are deleted. As a result of these selective processes, the repertoire of binding specificities in an individual sample can provide a history of past antigenic exposures, as well as being informative of inherent repertoire capabilities and limitations.

Adaptive immunological receptors of interest include immunoglobulins, or antibodies. This repertoire is highly plastic and can be directed to create antibodies with broad chemical diversity and high selectivity. There is also a good understanding of the potential diversity available and the mechanistic aspects of how this diversity is generated. Antibodies are composed of two types of chains (heavy and light), each containing a highly diversified antigen-binding domain (variable). The V, D, and J gene segments of the antibody heavy-chain variable genes go through a series of recombination events to generate a new heavy-chain gene. Antibodies are formed by a mixture of recombination among gene segments, sequence diversification at the junctions of these segments, and point mutations throughout the gene. The mechanisms are reviewed, for example in Maizels (2005) Annu. Revu. Genet. 39:23-46; Jones and Gellert (2004) Immunol. Rev. 200:233-248; Winter and Gearhart (1998) Immunol. Rev. 162:89-96.

Another adaptive immunological receptor of interest is the T-cell antigen receptor (TCR), which is a heterodimer of two chains, each of which is a member of the immunoglobulin superfamily, possessing an N-terminal variable (V) domain, and a C terminal constant domain. The variable domain of the TCR α-chain and β-chain has three hypervariable or complementarity determining regions (CDRs). The β-chain has an additional area of hypervariability (HV4) that does not normally contact antigen. Processes for generating diversity of the TCR are similar to those described for immunoglobulins. The TCR alpha chain is generated by VJ recombination, while the beta chain is generated by V(D)J recombination. Similarly, generation of the TCR gamma chain involves VJ recombination, while generation of the TCR delta chain occurs by V(D)J recombination. The intersection of these specific regions (V and J for the alpha or gamma chain, V D and J for the beta or delta chain) corresponds to the CDR3 region that is important for antigen- MHC recognition. It is the unique combination of the segments at this region, along with palindromic and random N- and P- nucleotide additions, which accounts for the TCR binding repertoire.

While reference is made to binding specificities, and indeed a good deal of serological analysis is based on the physical interactions between antigen and receptor, the underlying cause of the diversity lies in the genetic sequences expressed by lymphocytes, which sequences reflect the myriad processes of recombination, mutation and selection that have acted on the cell.

Georgiou et al. (Nature Biotechnology 32, pp. 158-168 (2014)) is a review article describing sequencing approaches for identifying the antibody repertoire in a sample.

WO 1997/24455 discloses a method for preparing cDNA that is complementary to the 5'-end of an RNA molecule. The method entails annealing a cDNA synthesis primer to the RNA molecule and synthesizing a first DNA strand, and further contacting the RNA molecule with a template switching oligonucleotide having a pre-selected arbitrary nucleotide sequence at its 5'-end and at least one riboguanine residue at its 3'-end. The template switching oligonucleotide attaches to the 5'-end of the RNA and serves as a template for extension of the 3'-end of the first DNA strand.

WO 2004/024953 discloses a method for preparation of cDNA tags for identifying expressed genes by providing cDNA-linker fragment complexes having recognition sites for various type II and IIS restriction enzymes.

Hug et al. (Journal of Theoretical Biology 221(4), pp. 615-624 (2003)) describes a theoretical approach for calculating the number of molecules of a single mRNA species in a complex mRNA sample. The approach is based in part on attaching random sequence tags to an individual mRNA species and determining the presence of each unique tag. The number of unique tags corresponds to the number of the single mRNA species in the original sample.

WO 2009/152928 also describes a method for the quantitative analysis of nucleic acids in a sample. The method includes steps of tagging the nucleic acids with random sequence tags and identifying the tags after an amplification step.

Methods of precisely determining the immune receptor repertoire of an individual, or a sample of interest from an individual, are of great interest for prognosis, diagnosis, and characterization.

### SUMMARY

The present invention provides methods for monitoring the immune repertoire. More specifically, the invention provides method of determining the immune repertoire in a subject, by isolating a plurality of RNA from a biological sample comprising a plurality of cell types obtained from a subject, producing immunoglobulin chain or TCR chain cDNAs from the RNA; adding a homopolymeric tail, a random molecular tag and a universal sequence to the 3' end of the cDNAs; amplifying the cDNAs by using a one or more immunoglobulin chain or TCR chain specific primers and a universal sequence specific primer comprising an flanking sequence specific to the sequencing platform to produce a plurality of molecular tagged immunoglobulin chain or TCR chain nucleic acids and sequencing the amplified immunoglobuling chain or TCR chain nucleic acids to produce a plurality of sequencing reads thereby determining the immune repertoire of the subject. Optionally, the method further includes a data analysis step such as grouping sequences reads with the same molecular tag and clustering sequences within the same group. In some aspects a consensus sequence for each cluster is determined to produce a collection of consensus sequences. The collection of consensus sequences is used to determine the diversity of the immune repertoire.

The molecular tag is an oligomer. The oligomer is at least a 9mer. The biological sample is blood or a fraction thereof. The blood is peripheral whole blood. The blood fraction comprises peripheral blood mononuclear cells. In some aspects the blood sample is sorted based upon extracellular or intracellular markers.

The immunoglobulin chain is the heavy chain or the light chain. The immunoglobulin heavy chain contains the immunoglobulin VDJ and constant regions. The TCR chain is the alpha chain, the beta chain, the gamma chain or the delta chain. The TCR chain contains the VJ and constant regions.

### BRIEF DESCRIPTION OF THE DRAWINGS

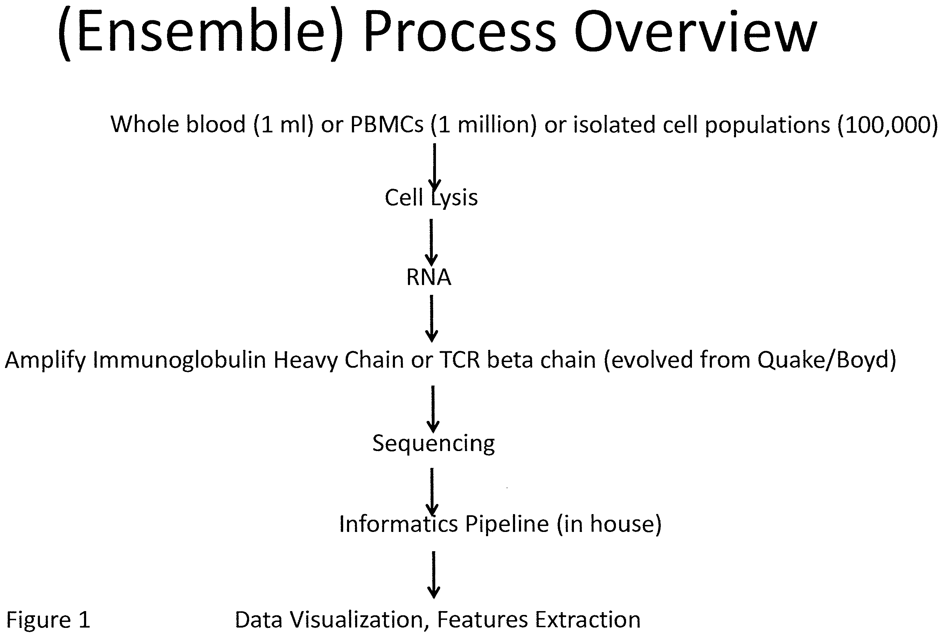

FIG. 1. is a schematic illustrating the method of the invention.
FIG. 2. is an illustration highlighting the differences of the method of the invention and multiplex sequencing of the immune repertoire.
FIG. 3 illustrates the elimination of capture and PCR bias by the method of the invention.
FIG. 4. is an illustration detailing the sequencing of the immune repertoire according to the method of the invention.
FIG. 5. is an illustration detailing the sequencing of the immune repertoire according to the method of the invention.
FIG. 6. is an illustration detailing the molecular tagging step of the sequencing of the immune repertoire according to the method of the invention.
FIG. 7. is a schematic illustrating the data analysis steps of the method of the invention.

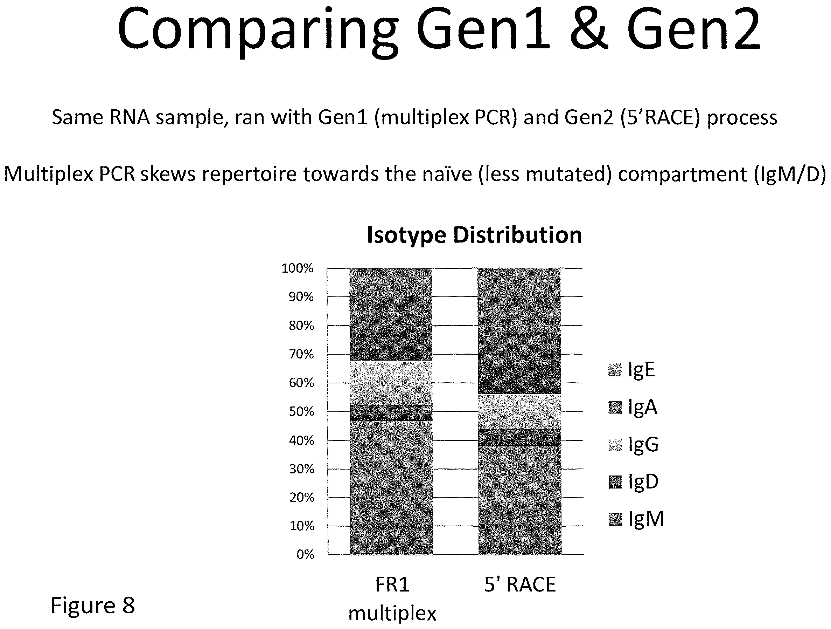

FIG. 8. is a graph showing the isotype distribution obtained using the multiplex PCR vs. the 5'RACE method of the invention from the same sample. The multiplex PCR skews the repertoire towards a more naive compartment.

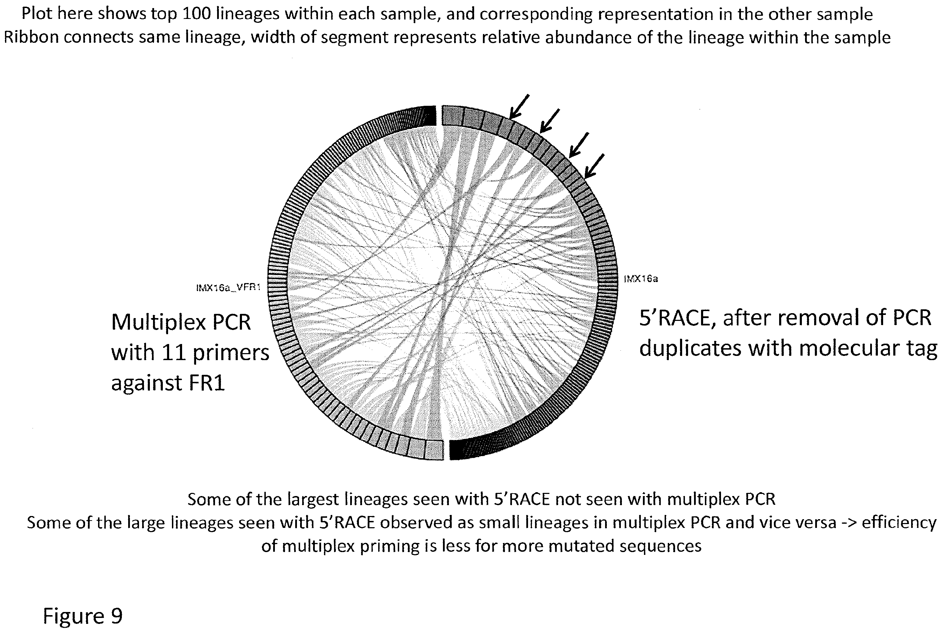

FIG. 9. is a plot showing the top 100 lineages within each sample and the corresponding resonation of the other sample. Ribbons connect the same lineage. Width of segment represents the relative abundance of the linage in the sample. A few large lineages seen with the method of the invention is absent in the same sample prepared by multiplex PCR.

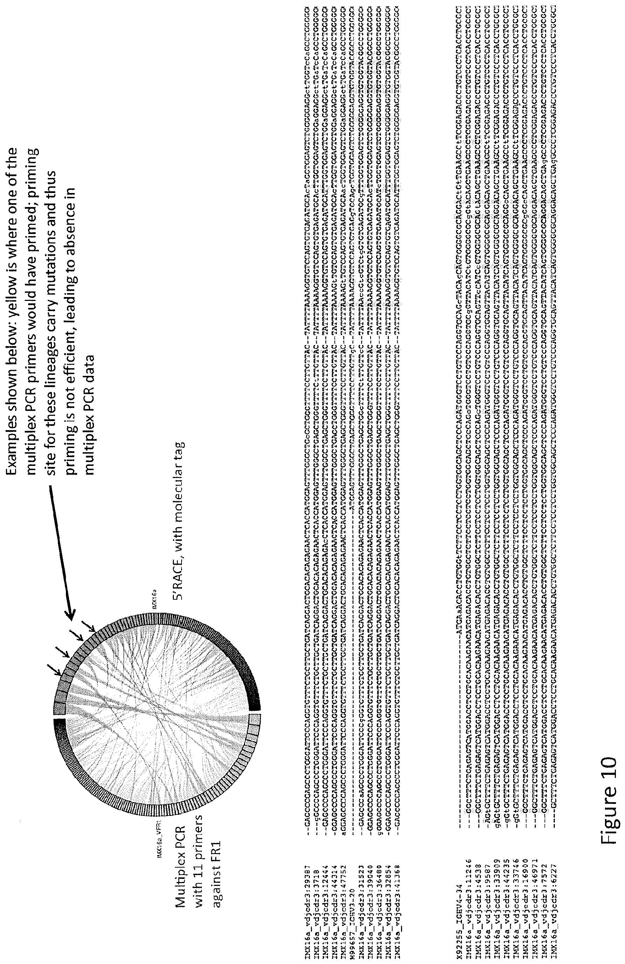

FIG. 10 is an illustration showing that multiplex PCR does not prime lineages with mutations, with the result being that highly mutated lineages are absent in from the multiplex PCR data set. The sequence identifiers of each of the sequences in FIG. 10 are SEQ ID NOs: 3-24 from top to bottom.

### DETAILED DESCRIPTION

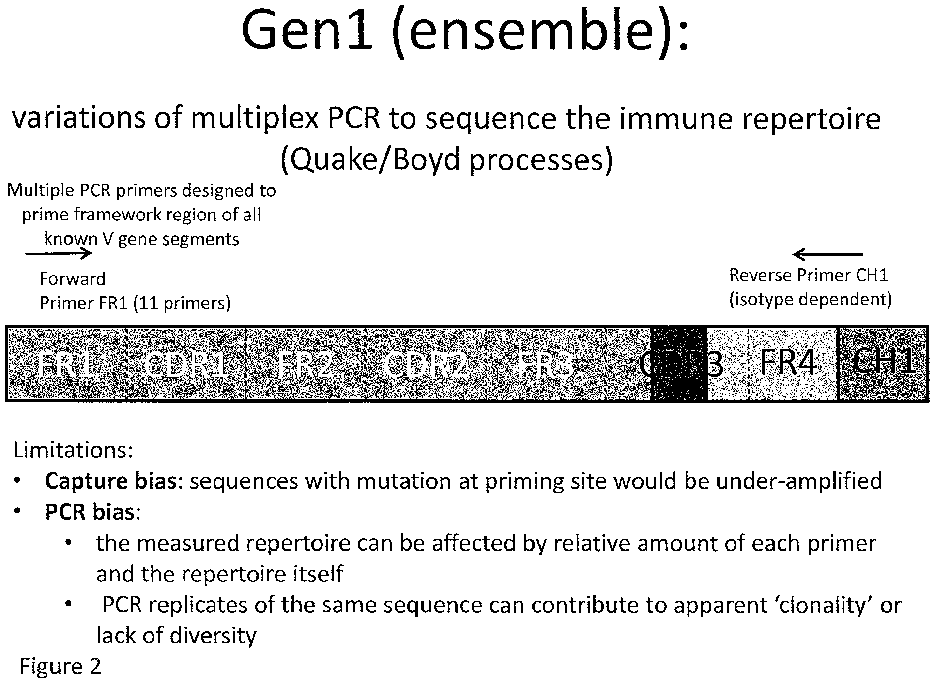

The present invention provides an improved method of sequencing the immune repertoire. Previous methods for determining the immune repertoire, such as those described in WO 2011/140433 and WO 2012/083069 are based upon multiplex PCR. Multiplex PCR has a number of limitations that make it particularly unsuited for accurately determining the immune repertoire. (See Figure 2) These limitations include capture bias and amplification bias owing to PCR.. Multiplex PCR techniques for sequencing the immune repertoire use primers designed to prime all framework regions of known V gene segments. When a mutation arises at the priming site capture bias occurs and the gene that had the mutation would be under-amplified. PCR bias results from unequal amplification of the genes due to either the relative amount of each primer and PCR replicates of the same sequence. Thus PCR bias can cause apparent clonality or a lack of diversity. Generally speaking, the observed repertoire is not a faithful, or linear representation of the actual underlying repertoire.

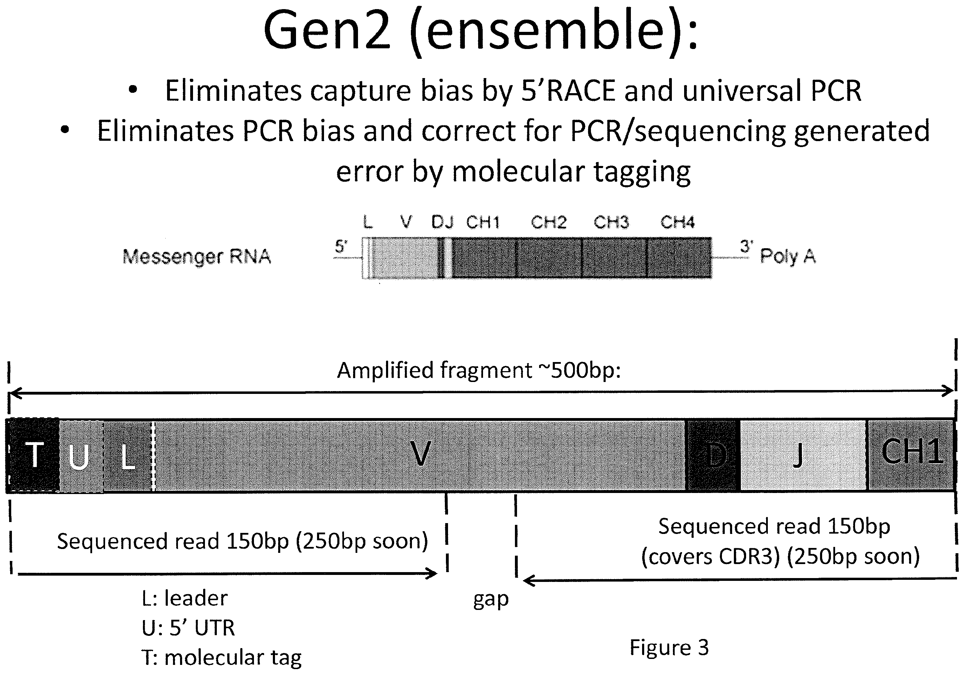

To eliminate capture bias the methods of the invention utilizes 5' RACE and universal PCR. PCR bias is eliminated by molecular tagging. (Figure 3)

Additionally, unlike previous methods of sequencing the immune repertoire which require the isolation of specific populations of immune cells (e.g., T-cells or B-cells), and the spatial isolation of such cells into individual cells and/or individual molecules of nucleic acid derived from such cells to form colonies, the present method sequences the immune repertoire directly from a heterogeneous nucleic acid mixture derived from a heterogeneous population of cells.

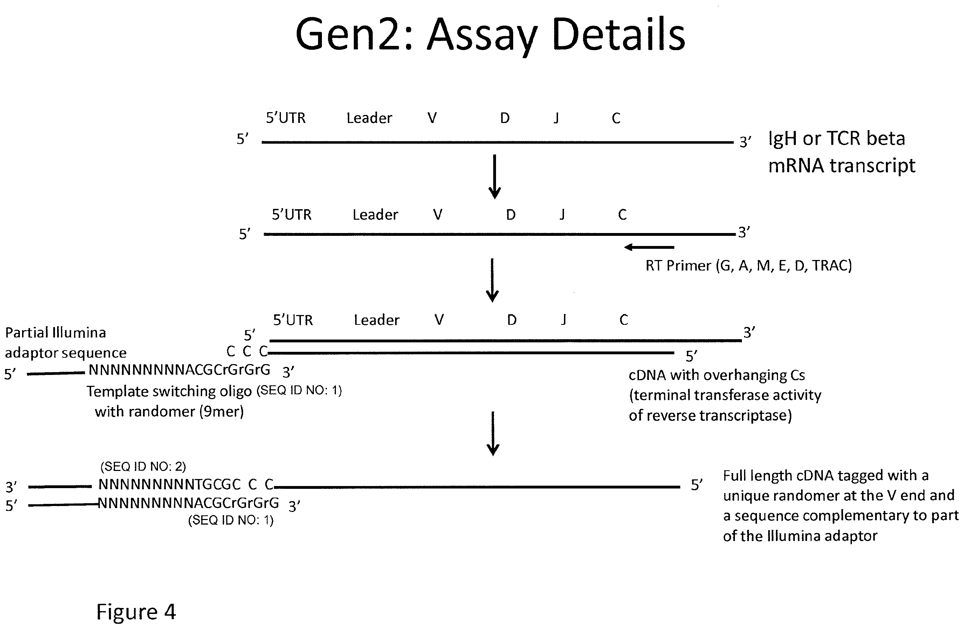

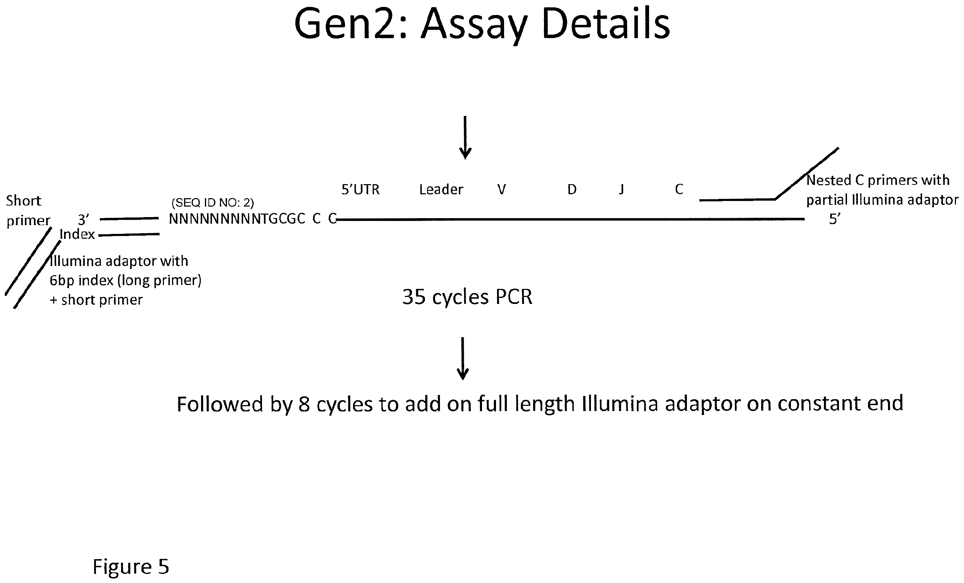

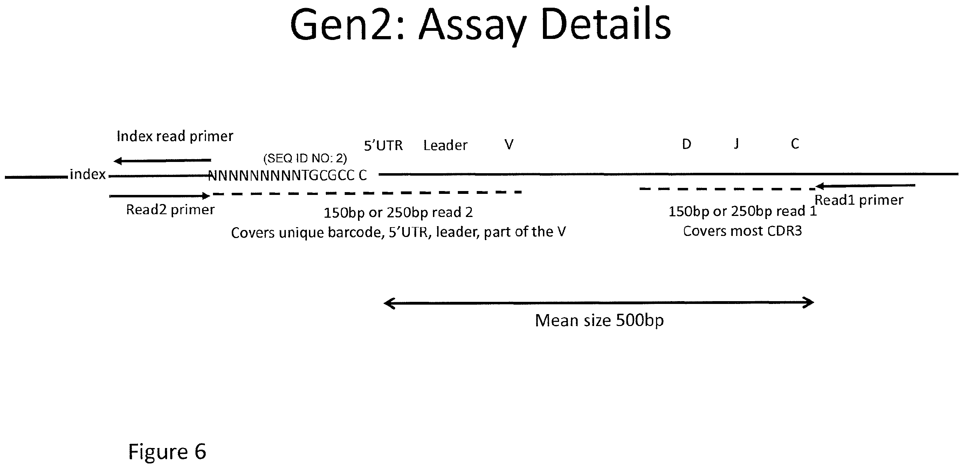

The methods of the invention generally involve the steps of obtaining a peripheral whole blood sample from a subject, isolating RNA from the peripheral whole blood sample, or fraction thereof (e.g., peripheral blood mononuclear cells), reverse transcribing the isolated RNA using immunoglobulin heavy chain or TCR beta chain specific primers to generate immunoglobulin (e.g., heavy chain or light chain) or TCR (e.g., alpha, beta, delta or gamma chain) cDNA transcripts. A short homopolymer is added to the end of the cDNA by the intrinsic property of reverse transcriptase. Oligonucleotides with 3' sequence complementary to the homopolymer and a 5' flanking sequence containing a universal sequence and molecular tag that is composed of random nucleotides would be used by the reverse transcriptase as template. The result is that the end of each cDNA molecule is extended with a short homopolymer, a unique molecular tag, and a universal sequence. This allows amplification of unknown sequences between the gene specific sequence and the 5'-end of the mRNA. (Figures 4-6). Because each cDNA molecule is labeled with a unique tag prior to amplification, the differential amplification of each cDNA molecule can be corrected for by counting each unique tag once, thereby providing a faithful measure of the abundance of each species in the repertoire. Sequence replicates of each cDNA molecule identified by the same molecular tag can be used to construct consensus sequences, therefore allowing correction for amplification and sequencing errors.

### Subjects

The methods of the invention utilize biological samples from subjects or individuals. The subject can be a patient, for example, a patient with an autoimmune disease, an infectious disease or cancer, or a transplant recipient. The subject can be a human or a non-human mammal. The subject can be a male or female subject of any age (e.g., a fetus, an infant, a child, or an adult).

### Samples

Samples used in the methods of the provided invention can include, for example, a bodily fluid from a subject, including amniotic fluid surrounding a fetus, aqueous humor, bile, blood and blood plasma, cerumen (earwax), Cowper's fluid or pre-ejaculatory fluid, chyle, chyme, female ejaculate, interstitial fluid, lymph, menses, breast milk, mucus (including snot and phlegm), pleural fluid, pus, saliva, sebum (skin oil), semen, serum, sweat, tears, urine, vaginal lubrication, vomit, feces, internal body fluids including cerebrospinal fluid surrounding the brain and the spinal cord, synovial fluid surrounding bone joints, intracellular fluid (the fluid inside cells), and vitreous humour (the fluids in the eyeball).

In one embodiment, the sample is a blood sample, such as a peripheral whole blood sample, or a fraction thereof. Preferably, the sample is whole, unfractionated blood.

The blood sample can be about 0.02, 0.03, 0.04, 0.05, 0.06, 0.07, 0.08 0.09, 0.1, 0.2, 0.3, 0.4, 0.5, 0.6, 0.7, 0.8, 0.9, 1.0, 1.5, 2.0, 2.5, 3.0, 3.5, 4.0, 4.5, or 5.0 mL.

The sample can be obtained by a health care provider, for example, a physician, physician assistant, nurse, veterinarian, dermatologist, rheumatologist, dentist, paramedic, or surgeon. The sample can be obtained by a research technician. More than one sample from a subject can be obtained.

The sample can include immune cells. The immune cells can include T-cells and/or B-cells. T-cells (T lymphocytes) include, for example, cells that express T-cell receptors. T-cells include Helper T-cells (effector T-cells or Th cells), cytotoxic T-cells (CTLs), memory T-cells, and regulatory T-cells. The sample can include a single cell in some applications (e.g., a calibration test to define relevant T-cells) or more generally at least 1,000, at least 10,000, at least 100,000, at least 250,000, at least 500,000, at least 750,000, or at least 1,000,000 T-cells.

B-cells include, for example, plasma B cells, memory B cells, B1 cells, B2 cells, marginal-zone B cells, and follicular B cells. B-cells can express immunoglobulins (antibodies, B cell receptor). The sample can include a single cell in some applications (e.g., a calibration test to define relevant B cells) or more generally at least 1,000, at least 10,000, at least 100,000, at least 250,000, at least 500,000, at least 750,000, or at least 1,000,000 B-cells.

The sample can include nucleic acid, for example, DNA (e.g., genomic DNA or mitochondrial DNA) or RNA (e.g., messenger RNA or microRNA). The nucleic acid can be cell-free DNA or RNA. In the methods of the provided invention, the amount of RNA or DNA from a subject that can be analyzed includes, for example, as low as a single cell in some applications (e.g., a calibration test) and as many as 10 millions of cells or more translating to a range of DNA of 6 pg-60 ug, and RNA of approximately 1 pg-10 ug.

### Amplification Reactions

Polymerase chain reaction (PCR) can be used to amplify the relevant regions from a collection of cells.

In some embodiments, the region to be amplified includes the full clonal sequence or a subset of the clonal sequence, including the V-D junction, D-J junction of an immunoglobulin or T-cell receptor gene, the full variable region of an immunoglobulin or T-cell receptor gene, the antigen recognition region, or a CDR, e.g., complementarity determining region 3 (CDR3).

In some embodiments, the immunoglobulin sequence is amplified using a primary and a secondary amplification step. Each of the different amplification steps can comprise different primers. The different primers can introduce sequence not originally present in the immune gene sequence. For example, the amplification procedure can add one or more tags to the 5' and/or 3' end of amplified immunoglobulin sequence. The tag can be a sequence that facilitates subsequent sequencing of the amplified DNA. The tag can be a sequence that facilitates binding the amplified sequence to a solid support. The tag can be a bar-code or label to facilitate identification of the amplified immunoglobulin sequence.

Other methods for amplification may not employ any primers in the V region. Instead, a specific primer can be used from the C segment and a generic primer can be put in the other side (5'). The generic primer can be appended in the cDNA synthesis through different methods including the well described methods of strand switching. Similarly, the generic primer can be appended after cDNA making through different methods including ligation.

Other means of amplifying nucleic acid that can be used in the methods of the invention include, for example, reverse transcription-PCR, real-time PCR, quantitative real-time PCR, digital PCR (dPCR), digital emulsion PCR (dePCR), clonal PCR, amplified fragment length polymorphism PCR (AFLP PCR), allele specific PCR, assembly PCR, asymmetric PCR (in which a great excess of primers for a chosen strand is used), colony PCR, helicase-dependent amplification (HDA), Hot Start PCR, inverse PCR (IPCR), in situ PCR, long PCR (extension of DNA greater than about 5 kilobases), multiplex PCR, nested PCR (uses more than one pair of primers), single-cell PCR, touchdown PCR, loop-mediated isothermal PCR (LAMP), and nucleic acid sequence based amplification (NASBA). Other amplification schemes include: Ligase Chain Reaction, Branch DNA Amplification, Rolling Circle Amplification, Circle to Circle Amplification, SPIA amplification, Target Amplification by Capture and Ligation (TACL) amplification, and RACE amplification.

The information in RNA in a sample can be converted to cDNA by using reverse transcription using techniques well known to those of ordinary skill in the art (see e.g., Sambrook, Fritsch and Maniatis, MOLECULAR CLONING: A LABORATORY MANUAL, 2nd edition (1989)). PolyA primers, random primers, and/or gene specific primers can be used in reverse transcription reactions.

Polymerases that can be used for amplification in the methods of the provided invention include, for example, Taq polymerase, AccuPrime polymerase, or Pfu. The choice of polymerase to use can be based on whether fidelity or efficiency is preferred.

After amplification of DNA from the genome (or amplification of nucleic acid in the form of cDNA by reverse transcribing RNA), the amplicons are directly sequenced.

### Sequencing

Any technique for sequencing nucleic acid known to those skilled in the art can be used in the methods of the provided invention. DNA sequencing techniques include classic dideoxy sequencing reactions (Sanger method) using labeled terminators or primers and gel separation in slab or capillary, sequencing-by-synthesis using reversibly terminated labeled nucleotides, pyrosequencing, 454 sequencing, allele specific hybridization to a library of labeled oligonucleotide probes, sequencing-by-synthesis using allele specific hybridization to a library of labeled clones that is followed by ligation, real time monitoring of the incorporation of labeled nucleotides during a polymerization step, and SOLiD sequencing.

In certain embodiments, the sequencing technique used in the methods of the provided invention generates at least 100 reads per run, at least 200 reads per run, at least 300 reads per run, at least 400 reads per run, at least 500 reads per run, at least 600 reads per run, at least 700 reads per run, at least 800 reads per run, at least 900 reads per run, at least 1000 reads per run, at least 5,000 reads per run, at least 10,000 reads per run, at least 50,000 reads per run, at least 100,000 reads per run, at least 500,000 reads per run, at least 1,000,000 reads per run, at least 2,000,000 reads per run, at least 3,000,000 reads per run, at least 4,000,000 reads per run at least 5000,000 reads per run, at least 6,000,000 reads per run at least 7,000,000 reads per run at least 8,000,000 reads per run, at least 9,000,000 reads per run, or at least 10,000,000 reads per run.

In some embodiments the number of sequencing reads per B cell sampled should be at least 2 times the number of B cells sampled, at least 3 times the number of B cells sampled, at least 5 times the number of B cells sampled, at least 6 times the number of B cells sampled , at least 7 times the number of B cells sampled, at least 8 times the number of B cells sampled, at least 9 times the number of B cells sampled, or at least at least 10 times the number of B cells The read depth allows for accurate coverage of B cells sampled, facilitates error correction, and ensures that the sequencing of the library has been saturated.

In some embodiments the number of sequencing reads per T-cell sampled should be at least 2 times the number of T-cells sampled, at least 3 times the number of T-cells sampled, at least 5 times the number of T-cells sampled, at least 6 times the number of T-cells sampled , at least 7 times the number of T-cells sampled, at least 8 times the number of T-cells sampled, at least 9 times the number of T-cells sampled, or at least at least 10 times the number of T-cells The read depth allows for accurate coverage of T-cells sampled, facilitates error correction, and ensures that the sequencing of the library has been saturated.

In certain embodiments, the sequencing technique used in the methods of the provided invention can generate about 30 bp, about 40 bp, about 50 bp, about 60 bp, about 70 bp, about 80 bp, about 90 bp, about 100 bp, about 110, about 120 by per read, about 150 bp, about 200 bp, about 250 bp, about 300 bp, about 350 bp, about 400 bp, about 450 bp, about 500 bp, about 550 bp, about 600 bp, about 700 bp, about 800 bp, about 900 bp, or about 1,000 by per read. For example, the sequencing technique used in the methods of the provided invention can generate at least 30, 40, 50, 60, 70, 80, 90, 100, 110, 120, 150, 200, 250, 300, 350, 400, 450, 500, 550, 600, 650, 700, 750, 800, 850, 900, 950, or 1,000 by per read.

### True Single Molecule Sequencing

A sequencing technique that can be used in the methods of the provided invention includes, for example, Helicos True Single Molecule Sequencing (tSMS) (Harris T. D. et al. (2008) Science 320:106-109). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm². The flow cell is then loaded into an instrument, e.g., HeliScope™. sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are detected by imaging the flow cell surface. After imaging, a cleavage step removes the fluorescent label, and the process is repeated with other fluorescently labeled nucleotides until the desired read length is achieved. Sequence information is collected with each nucleotide addition step.

### 454 Sequencing

Another example of a DNA sequencing technique that can be used in the methods of the provided invention is 454 sequencing (Roche) (Margulies, M et al. 2005, Nature, 437, 376-380). 454 sequencing involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated.

Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed.

### Genome Sequencer FLX™

Another example of a DNA sequencing technique that can be used in the methods of the invention is the Genome Sequencer FLX systems (Roche/454). The Genome Sequences FLX systems (e.g., GS FLX/FLX+, GS Junior) offer more than 1 million high-quality reads per run and read lengths of 400 bases. These systems are ideally suited for de novo sequencing of whole genomes and transcriptomes of any size, metagenomic characterization of complex samples, or resequencing studies.

### SOLiD™ Sequencing

Another example of a DNA sequencing technique that can be used in the methods of the provided invention is SOLiD technology (Life Technologies, Inc.). In SOLiD sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide.

The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

### Ion Torrent™ Sequencing

Another example of a DNA sequencing technique that can be used in the methods of the provided invention is the IonTorrent system (Life Technologies, Inc.). Ion Torrent uses a high-density array of micro-machined wells to perform this biochemical process in a massively parallel way. Each well holds a different DNA template. Beneath the wells is an ion-sensitive layer and beneath that a proprietary Ion sensor. If a nucleotide, for example a C, is added to a DNA template and is then incorporated into a strand of DNA, a hydrogen ion will be released. The charge from that ion will change the pH of the solution, which can be detected by the proprietary ion sensor. The sequencer will call the base, going directly from chemical information to digital information. The Ion Personal Genome Machine (PGM™) sequencer then sequentially floods the chip with one nucleotide after another. If the next nucleotide that floods the chip is not a match, no voltage change will be recorded and no base will be called. If there are two identical bases on the DNA strand, the voltage will be double, and the chip will record two identical bases called. Because this is direct detection-no scanning, no cameras, no light-each nucleotide incorporation is recorded in seconds.

### HiSeq™ and MiSeq™ Sequencing

Additional examples of sequencing technologies that can be used in the methods of the invention include the HiSEQ™ system (e.g., HiSEQ2000™ and HiSEQ1000™) and the MiSEQ™ system from Illumina, Inc. The HiSEQ™ system is based on massively parallel sequencing of millions of fragments using attachment of randomly fragmented genomic DNA to a planar, optically transparent surface and solid phase amplification to create a high density sequencing flow cell with millions of clusters, each containing about 1,000 copies of template per sq. cm. These templates are sequenced using four-color DNA sequencing-by-synthesis technology. The MiSEQ™ system uses TruSeq, Illumina's reversible terminator-based sequencing-by-synthesis.

### SOLEXA™ Sequencing

Another example of a sequencing technology that can be used in the methods of the invention is SOLEXA sequencing (Illumina). SOLEXA sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated.

### SMRT™ Sequencing

Another example of a sequencing technology that can be used in the methods of the provided invention includes the single molecule, real-time (SMRT™) technology of Pacific Biosciences. In SMRT™, each of the four DNA bases is attached to one of four different fluorescent dyes. These dyes are phospholinked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in and out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated.

### Nanopore Sequencing

Another example of a sequencing technique that can be used in the methods of the provided invention is nanopore sequencing (Soni G V and Meller A. (2007) Clin Chem 53: 1996-2001). A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence.

### Chemical-Sensitive Field Effect Transistor Array Sequencing

Another example of a sequencing technique that can be used in the methods of the provided invention involves using a chemical-sensitive field effect transistor (chemFET) array to sequence DNA (for example, as described in US Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

### Sequencing with an Electron Microscope

Another example of a sequencing technique that can be used in the methods of the provided invention involves using a electron microscope (Moudrianakis E. N. and Beer M. Proc Natl Acad Sci USA. 1965 March; 53:564-71). In one example of the technique, individual DNA molecules are labeled using metallic labels that are distinguishable using an electron microscope. These molecules are then stretched on a flat surface and imaged using an electron microscope to measure sequences.

Any one of the sequencing techniques described herein can be used in the methods of the invention.

### Digital Counting and Analysis

Sequencing allows for the presence of multiple immunoglobulin gene to be detected and quantified in a heterogeneous biological sample.

The high throughput sequencing provides a very large dataset, which is then analyzed in order to establish the repertoire.

High-throughput analysis can be achieved using one or more bioinformatics tools, such as ALLPATHS (a whole genome shotgun assembler that can generate high quality assemblies from short reads), Arachne (a tool for assembling genome sequences from whole genome shotgun reads, mostly in forward and reverse pairs obtained by sequencing cloned ends, BACCardl (a graphical tool for the validation of genomic assemblies, assisting genome finishing and intergenome comparison), CCRaVAT & QuTie (enables analysis of rare variants in large-scale case control and quantitative trait association studies), CNV-seq (a method to detect copy number variation using high throughput sequencing), Elvira (a set of tools/procedures for high throughput assembly of small genomes (e.g., viruses)), Glimmer (a system for finding genes in microbial DNA, especially the genomes of bacteria, archaea and viruses), gnumap (a program designed to accurately map sequence data obtained from next-generation sequencing machines), Goseq (an R library for performing Gene Ontology and other category based tests on RNA-seq data which corrects for selection bias), ICAtools (a set of programs useful for medium to large scale sequencing projects), LOCAS, a program for assembling short reads of second generation sequencing technology, Maq (builds assembly by mapping short reads to reference sequences, MEME (motif-based sequence analysis tools, NGSView (allows for visualization and manipulation of millions of sequences simultaneously on a desktop computer, through a graphical interface, OSLay (Optimal Syntenic Layout of Unfinished Assemblies), Perm (efficient mapping for short sequencing reads with periodic full sensitive spaced seeds, Projector (automatic contig mapping for gap closure purposes), Qpalma (an alignment tool targeted to align spliced reads produced by sequencing platforms such as Illumina, Solexa, or 454), RazerS (fast read mapping with sensitivity control), SHARCGS (SHort read Assembler based on Robust Contig extension for Genome Sequencing; a DNA assembly program designed for *de novo* assembly of 25-40mer input fragments and deep sequence coverage), Tablet (next generation sequence assembly visualization), and Velvet (sequence assembler for very short reads).

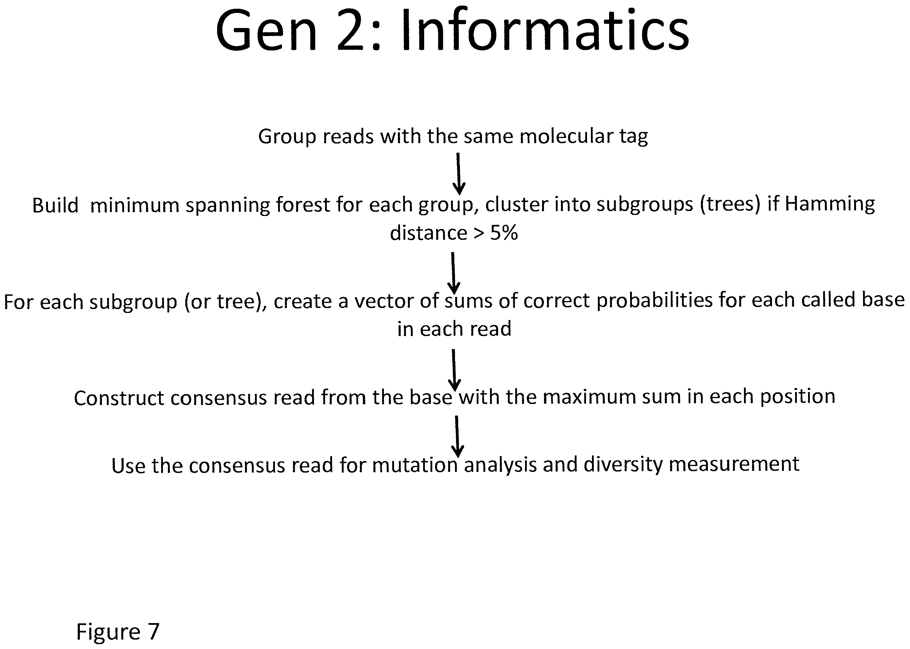

A Non-limiting example of data analysis steps are summarized in the flow chart of Figure 7.

*Grouping reads with the same molecular tag:* Initially sequences are matched based on identical molecular tag.

*Build a minimum spanning forest for each group*: Cluster into sungroups (tress) if Hamming distance is greater than 5%.

*For each subgroup (or tree), create a vector of sums of correct probabilities for each called base in each read.*

*Construct a consensus read from the base with the maximum sum in each position:* Consensus reads are used for mutation analysis and diversity measurement.

VDJ lineage diversity: VDJ usage is enumerated by the number of observed lineages falling into each VJ, VDJ, VJC, or VDJC (e.g., VDJ) combination at a given read- depth.

VDJ and unique sequence abundance histograms: Histograms are plotted by binning VDJ and unique sequence abundances (the latter which is either clustered or has undergone lineage-analysis filtering and grouping) into log-spaced bins.

3D representation of VJ, VDJ, VJC, or VDJC (e.g., VDJ) usage: Repertoires are represented by applying V-, D-, J-, and/or C- segments to different axes on a three-dimensional plot. Using either abundance (generally read number, which can be bias-normalized) or observed lineage diversity, bubbles of varying sizes are used at each V/D/J/C coordinate to represent the total usage of that combination.

Mutation vs. sequence abundance plots: After undergoing lineage analysis, unique sequences are binned by read-number (or bias-normalized abundance) into log-spaced bins. For a given abundance-bin, the number of mutations per unique sequence is averaged, giving a mutation vs. abundance curve.

Correlative measures of V, D, J, C, VJ, VDJ, VJC, VDJC, antibody heavy chain, antibody light chain, CDR3, or T-cell receptor usage (Pearson, KL divergence): VJ, VDJ, VJC, or VDJC (e.g., VDJ) combinations are treated as vectors with indexed components v,, weighted by either lineage-diversity or abundance for that VDJ combination. Pearson correlations and KL-divergences between each pair of individuals are then calculated over the indices.

The results of the analysis may be referred to herein as an immune repertoire analysis result, which may be represented as a dataset that includes sequence information, representation of V, D, J, C, VJ, VDJ, VJC, VDJC, antibody heavy chain, antibody light chain, CDR3, or T-cell receptor usage, representation for abundance of V, D, J, C, VJ, VDJ, VJC, VDJC, antibody heavy chain, antibody light chain, CDR3, or T-cell receptor and unique sequences; representation of mutation frequency, correlative measures of VJ V, D, J, C, VJ, VDJ, VJC, VDJC, antibody heavy chain, antibody light chain, CDR3, or T-cell receptor usage, etc. Such results may then be output or stored, e.g. in a database of repertoire analyses, and may be used in comparisons with test results, reference results, and the like.

After obtaining an immune repertoire analysis result from the sample being assayed, the repertoire can be compared with a reference or control repertoire to make a diagnosis, prognosis, analysis of drug effectiveness, or other desired analysis. A reference or control repertoire may be obtained by the methods of the invention, and will be selected to be relevant for the sample of interest. A test repertoire result can be compared to a single reference/control repertoire result to obtain information regarding the immune capability and/or history of the individual from which the sample was obtained. Alternately, the obtained repertoire result can be compared to two or more different reference/control repertoire results to obtain more in-depth information regarding the characteristics of the test sample. For example, the obtained repertoire result may be compared to a positive and negative reference repertoire result to obtain confirmed information regarding whether the phenotype of interest. In another example, two "test" repertoires can also be compared with each other. In some cases, a test repertoire is compared to a reference sample and the result is then compared with a result derived from a comparison between a second test repertoire and the same reference sample.

Determination or analysis of the difference values, i.e., the difference between two repertoires can be performed using any conventional methodology, where a variety of methodologies are known to those of skill in the array art, e.g., by comparing digital images of the repertoire output, by comparing databases of usage data, etc.

A statistical analysis step can then be performed to obtain the weighted contribution of the sequence prevalence, e.g. V, D, J, C, VJ, VDJ, VJC, VDJC, antibody heavy chain, antibody light chain, CDR3, or T-cell receptor usage, mutation analysis, etc. For example, nearest shrunken centroids analysis may be applied as described in Tibshirani et at. (2002) P.N.A.S. 99:6567-6572 to compute the centroid for each class, then compute the average squared distance between a given repertoire and each centroid, normalized by the within- class standard deviation.

A statistical analysis may comprise use of a statistical metric (e.g., an entropy metric, an ecology metric, a variation of abundance metric, a species richness metric, or a species heterogeneity metric.) in order to characterize diversity of a set of immunological receptors. Methods used to characterize ecological species diversity can also be used in the present invention. See, e.g., Peet, Annu Rev. Ecol. Syst. 5:285 (1974). A statistical metric may also be used to characterize variation of abundance or heterogeneity. An example of an approach to characterize heterogeneity is based on information theory, specifically the Shannon-Weaver entropy, which summarizes the frequency distribution in a single number. See, e.g., Peet, Annu Rev. Ecol. Syst. 5:285 (1974).

The classification can be probabilistically defined, where the cut-off may be empirically derived. In one embodiment of the invention, a probability of about 0.4 can be used to distinguish between individuals exposed and not-exposed to an antigen of interest, more usually a probability of about 0.5, and can utilize a probability of about 0.6 or higher. A "high" probability can be at least about 0.75, at least about 0.7, at least about 0.6, or at least about 0.5. A "low" probability may be not more than about 0.25, not more than 0.3, or not more than 0.4. In many embodiments, the above-obtained information is employed to predict whether a host, subject or patient should be treated with a therapy of interest and to optimize the dose therein.

### Diagnostics and Prognostics

The invention finds use in the prevention, treatment, detection, diagnosis, prognosis, or research into any condition or symptom of any condition, including cancer, inflammatory diseases, autoimmune diseases, allergies and infections of an organism. The organism is preferably a human subject but can also be derived from non-human subjects, e.g., non- human mammals. Examples of non-human mammals include, but are not limited to, non- human primates (e.g., apes, monkeys, gorillas), rodents (e.g., mice, rats), cows, pigs, sheep, horses, dogs, cats, or rabbits.

Examples of cancer include prostrate, pancreas, colon, brain, lung, breast, bone, and skin cancers. Examples of inflammatory conditions include irritable bowel syndrome, ulcerative colitis, appendicitis, tonsilitis, dermatitis. Examples of atopic conditions include allergy, asthma, etc.. Examples of autoimmune diseases include IDDM, RA, MS, SLE, Crohn's disease, Graves' disease, etc. Autoimmune diseases also include Celiac disease, and dermatitis herpetiformis. For example, determination of an immune response to cancer antigens, autoantigens, pathogenic antigens, vaccine antigens, and the like is of interest.

In some cases, nucleic acids (e.g., genomic DNA, mRNA, etc.) are obtained from an organism after the organism has been challenged with an antigen (e.g., vaccinated). In other cases, the nucleic acids are obtained from an organism before the organism has been challenged with an antigen (e.g., vaccinated). Comparing the diversity of the immunological receptors present before and after challenge, may assist the analysis of the organism's response to the challenge.

Methods are also provided for optimizing therapy, by analyzing the immune repertoire in a sample, and based on that information, selecting the appropriate therapy, dose, treatment modality, etc. that is optimal for stimulating or suppressing a targeted immune response, while minimizing undesirable toxicity. The treatment is optimized by selection for a treatment that minimizes undesirable toxicity, while providing for effective activity. For example, a patient may be assessed for the immune repertoire relevant to an autoimmune disease, and a systemic or targeted immunosuppressive regimen may be selected based on that information.

A signature repertoire for a condition can refer to an immune repertoire result that indicates the presence of a condition of interest. For example a history of cancer (or a specific type of allergy) may be reflected in the presence of immune receptor sequences that bind to one or more cancer antigens. The presence of autoimmune disease may be reflected in the presence of immune receptor sequences that bind to autoantigens. A signature can be obtained from all or a part of a dataset, usually a signature will comprise repertoire information from at least about 100 different immune receptor sequences, at least about 10² different immune receptor sequences, at least about 10³ different immune receptor sequences, at least about 10⁴ different immune receptor sequences, at least about 10⁵ different immune receptor sequences, or more. Where a subset of the dataset is used, the subset may comprise, for example, alpha TCR, beta TCR, MHC, IgH, IgL, or combinations thereof.

The classification methods described herein are of interest as a means of detecting the earliest changes along a disease pathway (e.g., a carcinogenesis pathway, inflammatory pathway, etc.), and/or to monitor the efficacy of various therapies and preventive interventions.

The methods disclosed herein can also be utilized to analyze the effects of agents on cells of the immune system. For example, analysis of changes in immune repertoire following exposure to one or more test compounds can performed to analyze the effect(s) of the test compounds on an individual. Such analyses can be useful for multiple purposes, for example in the development of immunosuppressive or immune enhancing therapies.

Agents to be analyzed for potential therapeutic value can be any compound, small molecule, protein, lipid, carbohydrate, nucleic acid or other agent appropriate for therapeutic use. Preferably tests are performed in vivo, e.g. using an animal model, to determine effects on the immune repertoire.

Agents of interest for screening include known and unknown compounds that encompass numerous chemical classes, primarily organic molecules, which may include organometallic molecules, genetic sequences, etc. An important aspect of the invention is to evaluate candidate drugs, including toxicity testing; and the like.

In addition to complex biological agents candidate agents include organic molecules comprising functional groups necessary for structural interactions, particularly hydrogen bonding, and typically include at least an amine, carbonyl, hydroxyl or carboxyl group, frequently at least two of the functional chemical groups. The candidate agents can comprise cyclical carbon or heterocyclic structures and/or aromatic or polyaromatic structures substituted with one or more of the above functional groups. Candidate agents can also be found among biomolecules, including peptides, polynucleotides, saccharides, fatty acids, steroids, purines, pyrimidines, derivatives, structural analogs or combinations thereof. In some instances, test compounds may have known functions (e.g., relief of oxidative stress), but may act through an unknown mechanism or act on an unknown target. Included are pharmacologically active drugs, genetically active molecules, etc. Compounds of interest include chemotherapeutic agents, hormones or hormone antagonists, etc. Exemplary of pharmaceutical agents suitable for this invention are those described in, "The Pharmacological Basis of Therapeutics," Goodman and Gilman, McGraw-Hill, New York, New York, (1996), Ninth edition, under the sections: Water, Salts and Ions; Drugs Affecting Renal Function and Electrolyte Metabolism; Drugs Affecting Gastrointestinal Function; Chemotherapy of Microbial Diseases; Chemotherapy of Neoplastic Diseases; Drugs Acting on Blood-Forming organs; Hormones and Hormone Antagonists; Vitamins, Dermatology; and Toxicology, all incorporated herein by reference. Also included are toxins, and biological and chemical warfare agents, for example see Somani, S.M. (Ed.), "Chemical Warfare Agents," Academic Press, New York, 1992).

Test compounds include all of the classes of molecules described above, and can further comprise samples of unknown content. Of interest are complex mixtures of naturally occurring compounds derived from natural sources such as plants, fungi, bacteria, protists or animals. While many samples will comprise compounds in solution, solid samples that can be dissolved in a suitable solvent may also be assayed. Samples of interest include environmental samples, e.g., ground water, sea water, mining waste, etc., biological samples, e.g. lysates prepared from crops, tissue samples, etc.; manufacturing samples, e.g. time course during preparation of pharmaceuticals; as well as libraries of compounds prepared for analysis; and the like (e.g., compounds being assessed for potential therapeutic value, i.e., drug candidates).

Samples or compounds can also include additional components, for example components that affect the ionic strength, pH, total protein concentration, etc. In addition, the samples may be treated to achieve at least partial fractionation or concentration. Biological samples may be stored if care is taken to reduce degradation of the compound, e.g. under nitrogen, frozen, or a combination thereof. The volume of sample used is sufficient to allow for measurable detection, for example from about 0.1 ml to 1 ml of a biological sample can be sufficient.

Compounds, including candidate agents, are obtained from a wide variety of sources including libraries of synthetic or natural compounds. For example, numerous means are available for random and directed synthesis of a wide variety of organic compounds, including biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

Some agent formulations do not include additional components, such as preservatives, that may have a significant effect on the overall formulation. Thus, such formulations consist essentially of a biologically active compound and a physiologically acceptable carrier, e.g. water, ethanol, DMSO, etc. However, if a compound is liquid without a solvent, the formulation may consist essentially of the compound itself.

### Databases of Expression Repertoires and Data Analysis

Also provided are databases of immune repertoires or of sets of immunological receptors. Such databases can typically comprise repertoires results derived from various individual conditions, such as individuals having exposure to a vaccine, to a cancer, having an autoimmune disease of interest, infection with a pathogen, and the like. Such databases can also include sequences of immunological receptors derived from synthetic libraries, or from other artificial methods. The repertoire results and databases thereof may be provided in a variety of media to facilitate their use. "Media" refers to a manufacture that contains the expression repertoire information of the present invention. The databases of the present invention can be recorded on computer readable media, e.g. any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. One of skill in the art can readily appreciate how any of the presently known computer readable mediums can be used to create a manufacture comprising a recording of the present database information. "Recorded" refers to a process for storing information on computer readable medium, using any such methods as known in the art. Any convenient data storage structure may be chosen, based on the means used to access the stored information. A variety of data processor programs and formats can be used for storage, e.g. word processing text file, database format, etc.

As used herein, "a computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

A variety of structural formats for the input and output means can be used to input and output the information in the computer-based systems of the present invention. Such presentation provides a skilled artisan with a ranking of similarities and identifies the degree of similarity contained in the test expression repertoire.

A scaled approach may also be taken to the data analysis. For example, Pearson correlation of the repertoire results can provide a quantitative score reflecting the signature for each sample. The higher the correlation value, the more the sample resembles a reference repertoire. A negative correlation value indicates the opposite behavior. The threshold for the classification can be moved up or down from zero depending on the clinical goal.

To provide significance ordering, the false discovery rate (FDR) may be determined.

First, a set of null distributions of dissimilarity values is generated. In one embodiment, the values of observed repertoires are permuted to create a sequence of distributions of correlation coefficients obtained out of chance, thereby creating an appropriate set of null distributions of correlation coefficients (see Tusher et al. (2001) PNAS 98, 51 18-21). The set of null distribution is obtained by: permuting the values of each repertoire for all available repertoires; calculating the pairwise correlation coefficients for all repertoire results; calculating the probability density function of the correlation coefficients for this permutation; and repeating the procedure for N times, where N is a large number, usually 300. Using the N distributions, one calculates an appropriate measure (mean, median, etc.) of the count of correlation coefficient values that their values exceed the value (of similarity) that is obtained from the distribution of experimentally observed similarity values at given significance level.

The FDR is the ratio of the number of the expected falsely significant correlations (estimated from the correlations greater than this selected Pearson correlation in the set of randomized data) to the number of correlations greater than this selected Pearson correlation in the empirical data (significant correlations). This cut-off correlation value may be applied to the correlations between experimental repertoires.

Using the aforementioned distribution, a level of confidence is chosen for significance. This is used to determine the lowest value of the correlation coefficient that exceeds the result that would have obtained by chance. Using this method, one obtains thresholds for positive correlation, negative correlation or both. Using this threshold(s), the user can filter the observed values of the pairwise correlation coefficients and eliminate those that do not exceed the threshold(s). Furthermore, an estimate of the false positive rate can be obtained for a given threshold. For each of the individual "random correlation" distributions, one can find how many observations fall outside the threshold range. This procedure provides a sequence of counts. The mean and the standard deviation of the sequence provide the average number of potential false positives and its standard deviation.

The data can be subjected to non-supervised hierarchical clustering to reveal relationships among repertoires. For example, hierarchical clustering may be performed, where the Pearson correlation is employed as the clustering metric. Clustering of the correlation matrix, e.g. using multidimensional scaling, enhances the visualization of functional homology similarities and dissimilarities. Multidimensional scaling (MDS) can be applied in one, two or three dimensions.

The analysis may be implemented in hardware or software, or a combination of both. In one embodiment of the invention, a machine-readable storage medium is provided, the medium comprising a data storage material encoded with machine readable data which, when using a machine programmed with instructions for using said data, is capable of displaying a any of the datasets and data comparisons of this invention. Such data may be used for .a variety of purposes, such as drug discovery, analysis of interactions between cellular components, and the like. In some embodiments, the invention is implemented in computer programs executing on programmable computers, comprising a processor, a data storage system (including volatile and non-volatile memory and/or storage elements), at least one input device, and at least one output device. Program code is applied to input data to perform the functions described above and generate output information. The output information is applied to one or more output devices, in known fashion. The computer may be, for example, a personal computer, microcomputer, or workstation of conventional design.

Each program can be implemented in a high level procedural or object oriented programming language to communicate with a computer system. However, the programs can be implemented in assembly or machine language, if desired. In any case, the language may be a compiled or interpreted language. Each such computer program can be stored on a storage media or device (e.g., ROM or magnetic diskette) readable by a general or special purpose programmable computer, for configuring and operating the computer when the storage media or device is read by the computer to perform the procedures described herein. The system may also be considered to be implemented as a computer- readable storage medium, configured with a computer program, where the storage medium so configured causes a computer to operate in a specific and predefined manner to perform the functions described herein. A variety of structural formats for the input and output means can be used to input and output the information in the computer-based systems of the present invention. One format for an output tests datasets possessing varying degrees of similarity to a trusted repertoire. Such presentation provides a skilled artisan with a ranking of similarities and identifies the degree of similarity contained in the test repertoire.

### Storing and Transmission of Data

Further provided herein is a method of storing and/or transmitting, via computer, sequence, and other, data collected by the methods disclosed herein. Any computer or computer accessory including, but not limited to software and storage devices, can be utilized to practice the present invention. Sequence or other data (e.g., immune repertoire analysis results), can be input into a computer by a user either directly or indirectly. Additionally, any of the devices which can be used to sequence DNA or analyze DNA or analyze immune repertoire data can be linked to a computer, such that the data is transferred to a computer and/or computer-compatible storage device. Data can be stored on a computer or suitable storage device (e.g., CD). Data can also be sent from a computer to another computer or data collection point via methods well known in the art (e.g., the internet, ground mail, air mail). Thus, data collected by the methods described herein can be collected at any point or geographical location and sent to any other geographical location

### Reagents and Kits

Also provided are reagents and kits thereof for practicing one or more of the above- described methods. The subject reagents and kits thereof may vary greatly. Reagents of interest include reagents specifically designed for use in production of the above described immune repertoire analysis. For example, reagents can include primer sets for cDNA synthesis, for PCR amplification and/or for high throughput sequencing of a class or subtype of immunological receptors. Gene specific primers and methods for using the same are described in U.S. Patent No. 5,994,076. The gene specific primer collections can include only primers for immunological receptors, or they may include primers for additional genes, e.g., housekeeping genes, controls, etc.

The kits of the subject invention can include the above described gene specific primer collections. The kits can further include a software package for statistical analysis, and may include a reference database for calculating the probability of a match between two repertoires. The kit may include reagents employed in the various methods, such as primers for generating target nucleic acids, dNTPs and/or rNTPs, which may be either premixed or separate, one or more uniquely labeled dNTPs and/or rNTPs, such as biotinylated or Cy3 or Cy5 tagged dNTPs, gold or silver particles with different scattering spectra, or other post synthesis labeling reagent, such as chemically active derivatives of fluorescent dyes, enzymes, such as reverse transcriptases, DNA polymerases, RNA polymerases, and the like, various buffer mediums, e.g. hybridization and washing buffers, prefabricated probe arrays, labeled probe purification reagents and components, like spin columns, etc., signal generation and detection reagents, e.g. streptavidin-alkaline phosphatase conjugate, chemifluorescent or chemiluminescent substrate, and the like.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed, site. Any convenient means may be present in the kits.

The above-described analytical methods may be embodied as a program of instructions executable by computer to perform the different aspects of the invention. Any of the techniques described above may be performed by means of software components loaded into a computer or other information appliance or digital device. When so enabled, the computer, appliance or device may then perform the above-described techniques to assist the analysis of sets of values associated with a plurality of genes in the manner described above, or for comparing such associated values. The software component may be loaded from a fixed media or accessed through a communication medium such as the internet or other type of computer network. The above features are embodied in one or more computer programs may be performed by one or more computers running such programs.

Software products (or components) may be tangibly embodied in a machine-readable medium, and comprise instructions operable to cause one or more data processing apparatus to perform operations comprising: a) clustering sequence data from a plurality of immunological receptors or fragments thereof; and b) providing a statistical analysis output on said sequence data. Also provided herein are software products (or components) tangibly embodied in a machine-readable medium, and that comprise instructions operable to cause one or more data processing apparatus to perform operations comprising: storing sequence data for more than 10², 10³, 10⁴, 10⁵, 10⁶, 10⁷, 10⁸, 10⁹, 10¹⁰, 10¹¹, or 10¹² immunological receptors or more than 10², 10³, 10⁴, 10⁵, 10⁶, 10⁷, 10⁸,10⁹, 10¹⁰, 10¹¹ , or 10¹² sequence reads.

In some examples, a software product (or component) includes instructions for assigning the sequence data into V, D, J, C, VJ, VDJ, VJC, VDJC, or VJ/VDJ lineage usage classes or instructions for displaying an analysis output in a multi-dimensional plot. In some cases, a multidimensional plot enumerates all possible values for one of the following: V, D, J, or C. (e.g., a three-dimensional plot that includes one axis that enumerates all possible V values, a second axis that enumerates all possible D values, and a third axis that enumerates all possible J values). In some cases, a software product (or component) includes instructions for identifying one or more unique patterns from a single sample correlated to a condition. The software product (or component) may also include instructions for normalizing for amplification bias. In some examples, the software product (or component) may include instructions for using control data to normalize for sequencing errors or for using a clustering process to reduce sequencing errors. A software product (or component) may also include instructions for using two separate primer sets or a PCR filter to reduce sequencing errors.

## Claims

1. A method of determining the immune repertoire in a subject, comprising:
a. isolating a plurality of RNA from a biological sample comprising a plurality of cell types obtained from a subject, wherein the RNA comprises immunoglobulin chain or TCR chain mRNA;
b. reverse transcribing the mRNA to produce immunoglobulin chain or TCR chain cDNA comprising a 3' homopolymer tail by using immunoglobulin chain or TCR chain specific primers and a reverse transcriptase;
c. adding a random molecular tag and a universal sequence to the 3' end of the immunoglobulin chain or TCR chain cDNA to form extended cDNA, by using the reverse transcriptase and oligonucleotides comprising a 3' sequence complementary to the homopolymer tail and a 5' flanking sequence comprising a universal sequence and a random molecular tag;
d. amplifying the extended cDNA by using one or more immunoglobulin chain or TCR chain specific primers and a universal sequence specific primer comprising a sequencing platform specific flanking sequence to produce a plurality of molecular tagged immunoglobulin chain or TCR chain nucleic acids;
e. sequencing the molecular tagged immunoglobulin chain or TCR chain nucleic acids to produce a plurality of sequencing reads thereby determining the immune repertoire of the subject.

2. The method of claim 1, further comprising a data analysis step after step (e).

3. The method of claim 2, wherein the data analysis comprises grouping sequences reads with the same molecular tag and clustering sequences within the same group to form clusters.

4. The method of claim 3, further comprising building a consensus sequence for each cluster to produce a collection of consensus sequences.

5. The method of claim 4, wherein the collection of consensus sequences is used to determine the diversity of the immune repertoire.

6. The method of claim 1, wherein the molecular tag is an oligomer.

7. The method of claim 6, wherein the oligomer is at least a 9mer.

8. The method of claim 1, wherein the biological sample is blood or a fraction thereof.

9. The method of claim 8, wherein the blood is peripheral whole blood.

10. The method of claim 8, wherein the blood fraction comprises peripheral blood mononuclear cells.

11. The method of claim 8, wherein the blood sample is sorted based upon extracellular or intracellular markers.

12. The method of claim 1, wherein the immunoglobulin chain is the heavy chain or the light chain.

13. The method of claim 12, wherein immunoglobulin heavy chain comprise the immunoglobulin VDJ and constant regions.

14. The method of claim 1, wherein the TCR chain is the alpha chain, the beta chain, the gamma chain or the delta chain.

15. The method of claim 14, wherein the TCR chain comprises the VJ and constant regions.

## Patentansprüche

1. Verfahren zur Bestimmung des Immunrepertoires in einem Subjekt, umfassend:
a. Isolieren einer Pluralität von RNA aus einer biologischen Probe umfassend eine von einem Subjekt erhaltene Pluralität von Zelltypen, wobei die RNA Immunglobulinketten- oder TCR-Ketten-mRNA umfasst;
b. reverse Transkription der mRNA, um Immunglobulinketten- oder TCR-Ketten-cDNA umfassend einen 3'-Homopolymerschwanz unter Verwendung von Immunglobulinketten- oder TCR-Ketten-spezifischen Primern und einer reversen Transkriptase zu erzeugen;
c. Hinzufügen eines zufälligen molekularen Tags und einer Universalsequenz zum 3'-Ende der Immunglobulinketten- oder TCR-Ketten-cDNA, um erweiterte cDNA zu bilden, unter Verwendung der reversen Transkriptase und von Oligonukleotiden umfassend eine 3'-Sequenz komplementär zum Homopolymerschwanz und eine 5'-Flankierungssequenz umfassend eine Universalsequenz und ein zufälliges molekulares Tag;
d. Amplifizieren der erweiterten cDNA unter Verwendung eines oder mehrerer Immunglobulinketten- oder TCR-Ketten-spezifischer Primer und eines Universalsequenz-spezifischen Primers umfassend eine Sequenzierungsplattform-spezifische Flankierungssequenz, um eine Pluralität von molekularen getaggten Immunglobulinketten- oder TCR-Ketten-Nukleinsäuren zu erzeugen;
e. Sequenzieren der molekularen getaggten Immunglobulinketten- oder TCR-Ketten-Nukleinsäuren, um eine Pluralität von Sequenzlesungen zu erzeugen, wodurch das Immunrepertoire des Subjekts bestimmt wird.

2. Verfahren nach Anspruch 1, ferner umfassend einen Datenanalyseschritt nach Schritt (e).

3. Verfahren nach Anspruch 2, wobei die Datenanalyse Gruppierungssequenzlesungen mit denselben molekularen Tag und Clustering-Sequenzen innerhalb derselben Gruppe zum Bilden von Clustern umfasst.

4. Verfahren nach Anspruch 3, ferner umfassend das Bauen einer Konsensussequenz für jeden Cluster, um eine Sammlung von Konsensussequenzen zu erzeugen.

5. Verfahren nach Anspruch 4, wobei die Sammlung der Konsensussequenzen benutzt wird, um die Diversität des Immunrepertoires zu bestimmen.

6. Verfahren nach Anspruch 1, wobei das molekulare Tag ein Oligomer ist.

7. Verfahren nach Anspruch 6, wobei das Oligomer mindestens ein 9mer ist.

8. Verfahren nach Anspruch 1, wobei die biologische Probe Blut oder eine Fraktion davon ist.

9. Verfahren nach Anspruch 8, wobei das Blut peripheres Vollblut ist.

10. Verfahren nach Anspruch 8, wobei die Blutfraktion mononukleäre Zellen des peripheren Bluts umfasst.

11. Verfahren nach Anspruch 8, wobei die Blutprobe auf Basis extrazellulärer oder intrazellulärer Marker sortiert wird.

12. Verfahren nach Anspruch 1, wobei die Immunglobulinkette die Schwerkette oder die Leichtkette ist.

13. Verfahren nach Anspruch 12, wobei die Immunglobulin-Schwerkette die Immunglobulin-VDJ- und -Konstantregionen umfasst.

14. Verfahren nach Anspruch 1, wobei die TCR-Kette die Alpha-Kette, die Beta-Kette, die Gamma-Kette oder die Delta-Kette ist.

15. Verfahren nach Anspruch 14, wobei die TCR-Kette die VJ- und Konstantregionen umfasst.

## Revendications

1. Méthode permettant de déterminer le répertoire immunitaire chez un patient, comprenant :
a. l'isolement d'une pluralité d'ARN à partir d'un échantillon biologique comprenant une pluralité de types de cellules obtenus à partir d'un sujet, ledit ARN comprenant un ARNm de chaîne d'immunoglobuline ou de chaîne de TCR ;
b. la transcription inverse de l'ARNm pour produire un ADNc de chaîne d'immunoglobuline ou de chaîne de TCR comprenant une queue homopolymère 3' au moyen d'amorces spécifiques à une chaîne d'immunoglobuline ou à une chaîne de TCR et d'une transcriptase inverse ;
c. l'ajout d'un marqueur moléculaire aléatoire et d'une séquence universelle à l'extrémité 3' de l'ADNc de chaîne d'immunoglobuline ou de chaîne de TCR pour former un ADNc allongé, au moyen de la transcriptase inverse et d'oligonucléotides comprenant une séquence 3' complémentaire à la queue homopolymère et une séquence flanquante 5' comprenant une séquence universelle et une marqueur moléculaire aléatoire ;
d. l'amplification de l'ADNc allongé au moyen d'une ou plusieurs amorces spécifiques à une chaîne d'immunoglobuline ou à une chaîne de TCR et d'une amorce spécifique à une séquence universelle comprenant une séquence flanquante spécifique à une plateforme de séquençage pour produire une pluralité d'acides nucléiques de chaîne d'immunoglobuline ou de chaîne de TCR à marquage moléculaire ;
e. le séquençage des acides nucléiques de chaîne d'immunoglobuline ou de chaîne de TCR à marquage moléculaire pour produire une pluralité de lectures de séquençage déterminant ainsi le répertoire immunitaire du sujet.

2. Méthode selon la revendication 1, comprenant en outre une étape d'analyse de données après l'étape (e).

3. Méthode selon la revendication 2, ladite analyse de données comprenant le groupement des lectures de séquences dotées du même marqueur moléculaire et le regroupement des séquences au sein du même groupe pour former des regroupements.

4. Méthode selon la revendication 3, comprenant en outre la construction d'une séquence consensus pour chaque regroupement pour produire une collection de séquences consensus.

5. Méthode selon la revendication 4, ladite collection de séquences consensus étant utilisée pour déterminer la diversité du répertoire immunitaire.

6. Méthode selon la revendication 1, ledit marqueur moléculaire étant un oligomère.

7. Méthode selon la revendication 6, ledit oligomère étant au moins un 9mère.

8. Méthode selon la revendication 1, ledit échantillon biologique étant du sang ou une fraction de celui-ci.

9. Méthode selon la revendication 8, ledit sang étant du sang total périphérique.

10. Méthode selon la revendication 8, ladite fraction sanguine comprenant des cellules mononucléaires de sang périphérique.

11. Méthode selon la revendication 8, ledit échantillon de sang étant trié en fonction des marqueurs extracellulaires et intracellulaires.

12. Méthode selon la revendication 1, ladite chaîne d'immunoglobuline étant la chaîne lourde ou la chaîne légère.

13. Méthode selon la revendication 12, ladite chaîne lourde d'immunoglobuline comprenant les régions constantes et VDJ d'immunoglobuline.

14. Méthode selon la revendication 1, ladite chaîne de TCR étant la chaîne alpha, la chaîne bêta, la chaîne gamma ou la chaîne delta.

15. Méthode selon la revendication 14, ladite chaîne de TCR comprenant les régions constantes et VJ.
